# EUROPEAN PATENT SPECIFICATION

(11) **EP 2 763 655 B1**
(45) Date of publication and mention of the grant of the patent: **22.06.2016**
(21) Application number: 13724470.3
(22) Date of filing: 08.05.2013
(51) Int. Cl.: A61L 29/08, A61L 29/16, A61L 31/10, A61L 31/16, A61K 31/4523, A61K 9/00, A61F 2/915, A61F 2/958, A61M 25/10, A61K 31/337, A61K 31/715, A61K 31/727

(54) **COATED MEDICAL DEVICES COMPRISING A WATER-INSOLUBLE THERAPEUTIC AGENT AND AN ADDITIVE**
BESCHICHTETES MEDIZINISCHES HILFSMITTEL ENTHALTEND EINEN WASSERUNLÖSLICHEN WIRKSTOFF UND EINEN ZUSÄTZLICHEN HILFSSTOFF
UN DISPOSITIF MÉDICINAL ENROBÉ CONTENANT UN AGENT THÉRAPEUTIQUE INSOLUBLE DANS L'EAU ET UN AGENT INACTIF

(30) Priority: 09.05.2012 US 201261644720 P; 15.03.2013 US 201313834339
(43) Date of publication of application: 13.08.2014
(73) Proprietor: Cook Medical Technologies LLC, Bloomington, IN 47402 (US)
(72) Inventor: LESLIE, Julia, E., League City, TX 77573 (US); SHIRLEY, Gary, B., Bloomington, IN 47403 (US); SARASAM, Aparna, R., San Jose, CA 95117 (US); GRABLE, Charity, West Lafayette, IN 74906 (US); BARNETT, Angela, R., Lafayette, IN 47904 (US)
(74) Representative: Simpson, Tobias Rutger
(86) International application number: PCT/US2013/040104
(87) International publication number: WO 2013/169879

(56) References cited:
- EP-A2- 1 987 851
- WO-A1-03/094991
- WO-A1-2009/051780
- WO-A2-2006/086693
- CA-A1- 2 517 702
- US-A1- 2011 218 611
- US-B1- 6 179 817
- DATABASE EPODOC EUROPEAN PATENT OFFICE, THE HAGUE, NL; 11 February 2009 (2009-02-11), CHEN J; HUANG N; LENG Y; LI Q; SUN H; WAN G; WANG J; WU X; YANG P; YOU T; ZHAO A: "Preparation method of blood vessel support or cardiac valve surface coating with good biocompatibility", XP002704081, Database accession no. CN-200810046051-A & CN 101 361 988 B (UNIV JIAOTONG SOUTHWEST [CN]) 21 December 2011 (2011-12-21)

## Description

### REFERENCE TO RELATED APPLICATION

This application claims the benefit of U.S. Provisional Application No. 61/644,720, filed May 9, 2012, and U.S. Application No. 13/834,339, filed March 15, 2013, which are both hereby incorporated by reference.

### BACKGROUND

The present invention relates generally to medical substances and devices, and methods for their preparation and use. In certain of its aspects, the present invention relates to medical devices that have an implantable structure and a coating layer comprising a water-insoluble therapeutic agent and one or more additives selected from heparin, heparan sulfate, dextran and dextran sulfate, and physiologically-acceptable salts thereof.

Local delivery of a therapeutic agent can be useful in the treatment of many medical conditions. Illustratively, local delivery of a therapeutic agent within a body vessel and/or to a selected portion of internal body tissue can eliminate or reduce the need for systemic delivery of the therapeutic agent thus minimizing any potential adverse effect of the therapeutic agent on areas of the body not needing treatment or on patient health in general.

Minimally invasive implantable medical devices, such as catheters and stents, can provide a platform for delivering therapeutic agents to internal body tissue. For example, balloon catheters and/or stents may be used to deliver a therapeutic agent directly to the target site within a body vessel such as an artery or vein. One example of a condition that can be beneficially treated by local administration of a therapeutic agent with a balloon catheter is the delivery of a therapeutic agent in combination with percutaneous transluminal angioplasty (PTA), a technique used to dilate stenotic portions of blood vessels. In such cases, a catheter balloon coated with the therapeutic agent can be positioned at a blocked lumen or target site during PTA, and the balloon is inflated causing dilation of the vessel lumen. The catheter balloon is pressed against the vessel wall for delivery of the therapeutic agent to the vessel wall. The balloon is deflated and the catheter is then removed from the target site and the patient's lumen thereby allowing blood to more freely flow through the now less restricted lumen.

WO2009/051780, US2011/218611, EP1987851, CA2517702, WO 2006/086693, WO03/094991, US6179817 as well as CHEN J; HUANG N; LENG Y; LI Q; SUN H; WAN G; WANG J; WU X; YANG P; YOU T; ZHAO A: "Preparation method of blood vessel support or cardiac valve surface coating with good biocompatibility", ; & CN 101 361 988 B (UNIV JIAOTONG SOUTHWEST [CN]) 21 December 2011 (2011-12-21) disclose implantable medical devices either having one coating or two coatings comprising a therapeutic agent and heparin/heparan.

Although PTA and related procedures aid in alleviating intraluminal constrictions, such constrictions or blockages may reoccur in many cases. The cause of these recurring obstructions, termed restenosis, may be due to the body responding to the surgical procedure. Restenosis of the vessel may develop over several months after the procedure, and may require another angioplasty procedure or a surgical bypass operation to correct. Proliferation and migration of smooth muscle cells (SMC) from the media layer of the lumen to the intimal layer cause an excessive production of extracellular matrix (ECM), which is believed to be one of the leading contributors to the development of restenosis. The extensive thickening of tissues narrows the lumen of the blood vessel, constricting or blocking the blood flow through the vessel. Therapeutic agents that inhibit restenosis may be locally delivered during PTA from a catheter and/or by placement of a stent configured to continue to release the therapeutic agent after the PTA procedure.

The delivery of the therapeutic agent from coatings in these and other minimally invasive procedures can be complicated by the need both to have a coating that is durable during delivery, but which effectively delivers the therapeutic agent when implanted in the region where local treatment is desired. Because natural biological environments are aqueous, it can occur that a coating containing a water-insoluble therapeutic agent is sufficiently durable during travel to the intended delivery site, but then fails to optimally deliver the therapeutic agent at the site. Needs thus exist for compositions, coatings, and coated implantable medical devices which enable the beneficial delivery of a water-insoluble therapeutic agent locally to a site intended for treatment.

### SUMMARY

In one aspect, the present invention provides a medical device comprising
an implantable medical device structure having a surface, and a coating layer carried by the surface. The coating layer includes (i) a water-insoluble therapeutic agent; and (ii) one or more additives selected from heparin, heparan sulfate dextran, and dextran sulfate, and physiologically-acceptable salts thereof according to claim 1. The implantable medical device structure can be an expandable structure such as a balloon or stent. The one or more additives can be present in an amount that is effective to modify the rate of release of the water-insoluble therapeutic agent by the coating layer. The water-insoluble therapeutic agent can be a restenosis inhibiting agent, which in certain embodiments is paclitaxel or a macrolide immunosuppressive agent such as sirolimus, pimecrolimus, tacrolimus, everolimus, zotarolimus, novolimus, myolimus, temsirolimus, deforolimus, or biolimus zotarolimus, sirolimus, pimecrolimus, biolimus tacrolimus, or everolimus.

In another aspect, the present invention provides a medical device that includes a catheter shaft and an inflatable balloon mounted on the catheter shaft. A coating layer is carried by the inflatable balloon and includes (i) a water-insoluble therapeutic agent; and (ii) one or more additives selected from heparin, heparan sulfate, dextran and dextran sulfate, and physiologically-acceptable salts thereof. The coating layer can include said one or more additives in an amount that is effective to increase the rate of release of the water-insoluble therapeutic agent from the coating layer. The coating layer can be adhered directly to a balloon wall of the inflatable balloon, or to another coating layer directly on or carried by a balloon wall of the balloon. The water-insoluble therapeutic agent can be a restenosis-inhibiting agent. The water-insoluble therapeutic agent can be included in the coating layer in a weight ratio according to claim 1. The water-insoluble therapeutic agent can be paclitaxel, and when so the coating layer can include the paclitaxel at a level of about 1 micrograms/mm² to about 10 micrograms/mm². When the coating layer includes paclitaxel at these levels or other levels, the coating layer can include heparin sodium at a level less than the paclitaxel, for example in the range of about 0.05 to about 2 micrograms/mm².

In another aspect, the present invention provides a medical device including an implantable medical device structure having a surface, and a coating layer carried by the surface. The coating layer includes (i) paclitaxel or a macrolide immunosuppressive agent, and (ii) at least one additive selected from heparin, heparan sulfate, dextran, and dextran sulfate, and physiologically-acceptable salts thereof. The implantable medical device structure can be an expandable structure such as a balloon or a stent. The coating layer can include said one or more additives in an amount that is effective to increase the rate of release of the water-insoluble therapeutic agent from the coating layer. The medical device can include the paclitaxel or macrolide immunosuppressive agent in the coating layer in a weight ratio according to claim 1. The one or more additives can include heparin or a physiologically-acceptable salt thereof having a weight average molecular weight in the range of about 2000 to about 40000 Daltons. The coating layer can include paclitaxel and heparin sodium in a weight ratio in the range of about 12:1 to about 7:1.

In another aspect, the present invention provides a method for manufacturing a medical device. The method includes applying a flowable medium comprising liquid, a water-insoluble therapeutic agent and one or more additives selected from heparin, heparan sulfate, dextran and dextran sulfate, and physiologically-acceptable salts thereof, to a surface of an implantable medical device structure or to a surface of a coating layer carried by the implantable medical device structure. The method also includes removing liquid from the medium to form a coating layer comprising the water-insoluble therapeutic agent and the one or more additives. The liquid of the flowable medium can include water and/or an organic solvent. The step of removing liquid can include evaporating liquid. The implantable medical device structure can be a balloon or a stent.

In another aspect, the present invention provides a coating layer including (i) a water-insoluble therapeutic agent and (ii) one or more additives selected from heparin, heparan sulfate, dextran and dextran sulfate, and physiologically acceptable salts thereof. The coating layer can be for delivery of the water-insoluble therapeutic agent from an implantable medical device structure. In such embodiments the implantable medical device structure can be configured for temporary or permanent implantation in a vascular vessel of a patient, and/or the water-insoluble therapeutic agent can be a restenosis-inhibiting agent. In certain embodiments, the water-insoluble therapeutic agent is paclitaxel and/or the one or more additives includes heparin or a physiologically-acceptable salt thereof. Where the coating layer includes paclitaxel and heparin sodium, it can include them in a weight ratio in the range of about 12:1 to about 7:1 relative to one another.

Still further aspects of the invention provide methods for treating a patient that include implanting in the patient a medical device or coating layer as specified above and/or elsewhere herein.

### BRIEF DESCRIPTION OF THE FIGURES

**Fig. 1** provides a perspective view of a therapeutic agent-delivering balloon catheter in accordance with one embodiment of the invention in an inflated condition.
**Fig. 2** provides a cross-sectional view of the balloon-mounted region of the balloon catheter of **Fig. 1** taken along a central longitudinal axis.
**Fig. 3** provides a cross-sectional view of the catheter shaft of the balloon catheter of **Fig. 1** taken along line 3-3 and viewed in the direction of the arrows.
**Fig. 4** provides a perspective view of the balloon catheter of **Fig. 1** in a folded condition.
**Fig. 5** provides a cross-sectional view of the balloon catheter of **Fig. 4** taken along line 5-5 and viewed in the direction of the arrows.
**Fig. 5a** provides a cross-sectional view illustrating an alternate coating pattern to that shown in **Fig. 5**.
**Fig. 5b** provides a cross-sectional view illustrating another alternate coating pattern to that shown in **Fig. 5**.
**Fig. 6** provides a cross-sectional view of the balloon catheter of **Fig. 1** taken along a longitudinal axis and illustrating an alternate coating configuration.
**Fig. 7** provides a cross-sectional view of the balloon catheter of **Fig. 1** taken along a longitudinal axis and illustrating another alternate coating configuration.
**Fig. 8** provides a perspective view of a therapeutic agent-delivering stent in accordance with one embodiment of the invention.
**Fig. 9** provides a perspective view of a coated therapeutic agent-delivering balloon catheter having a coated balloon-expandable stent mounted thereon in accordance with an embodiment of the invention.
**Fig. 10** provides a side view of a therapeutic agent-delivering scoring balloon catheter in accordance with one embodiment of the invention in an inflated condition.
**Fig. 11** provides an enlarged cross-sectional view of a dilation element of the scoring balloon catheter of **Fig. 10** and adjacent balloon wall film portions.

### DETAILED DESCRIPTION

As disclosed above, in certain aspects the present invention relates to medical devices carrying a coating layer including (i) a water-insoluble therapeutic agent and (ii) one or more additives selected from heparin, heparan sulfate, dextran, dextran sulfate, and physiologically-acceptable salts of any of these (such a coating layer hereinafter sometimes referred to as a "Therapeutic Agent Release Layer" or "TA Release Layer"), and methods for the preparation and use of such medical devices. In the discussions that follow, a number of potential features or selections of the water-insoluble therapeutic agent, heparin or salts thereof, heparan sulfate or salts thereof, dextran, dextran sulfate or salts thereof, implantable medical device structure, or other aspects, are disclosed. It is to be understood that each such disclosed feature or features can be combined with the generalized features discussed in the Summary above, to form a disclosed embodiment of the present invention.

The medical device may be any of a wide variety of devices having an implantable medical device structure adapted for temporary or permanent implantation in a human or veterinary patient. Medical devices having structures implantable in a bodily passage will often be used. The bodily passage may for example be a passage of the alimentary system, the urogenital system, the biliary system, or the cardiovascular system. Medical devices including a device structure implantable in the cardiovascular system are preferred, including for example those implantable in a vessel or chamber of the cardiovascular system of a human or animal patient through which blood travels. The passage may for example be a tubular passage such as an artery or vein, or may be a larger chamber such as a ventricle or atrium of the heart. Implantable medical devices that include structures that span or bridge between cardiovascular or other bodily passages are also contemplated. The implantable medical device can be adapted to be entirely or only partially implanted in a cardiovascular passage or other bodily passage.

By way of example, the medical device can be or include a catheter, a wire guide, a stent, a coil, a needle, a graft, a filter, a balloon, a cutting balloon, a scoring balloon, or any combination of these. Suitable filters include for example vena cava filters such as the Cook Celect® and Cook Günther Tulip® and Cook Gianturco-Roehm Bird's Nest® filters available from Cook Incorporated, Bloomington Indiana, USA. Suitable stents include those without a sheath covering, for example the Cook Zilver® stent available from Cook Incorporated. Suitable stents also include those with a sheath covering. Suitable coils include embolization coils. Suitable wire guides include for instance traditional wire guides as well as wire guides with an attached expandable structure for expansion within a blood vessel lumen, such as a coil, where the expandable structure can optionally carry the coating or coatings as disclosed herein. These or other implants, in certain preferred embodiments, have at least a portion that is configured to expand during deployment so as to contact walls of the passage in which they are implanted to anchor within the passage. In this regard, both self-expanding and force-expandable (e.g. balloon-expandable) stents or other implantable medical devices are contemplated as being within the scope of embodiments of the present invention. As well, it is contemplated that the implantable medical device may be configured for introduction by a minimally-invasive surgical technique, especially percutaneous introduction, or may be configured for introduction by invasive surgery e.g. in which the site of intended implantation in the blood passage is surgically exposed from the exterior of the patient for introduction of the implantable medical device. The implantable medical device may also be percutaneously retrievable, for example a percutaneously retrievable stent, filter or frame (e.g. a spiral frame). These and other variations in the implantable medical device and its associated procedure for introduction will be apparent to those skilled in the pertinent art from the descriptions herein.

The implantable medical device can be made from any suitable material or combination of materials. Illustratively, the implantable medical device can include a metal such as stainless steel, tantalum, titanium, nitinol, cobalt, chromium, nickel,, molybdenum, manganese, gold, platinum, inconel, iridium, silver, tungsten, elgiloy, alloys of any of these, ferrous alloys, palladium alloys, rhenium alloys, or another biocompatible metal; carbon or carbon fiber; a calcium-containing inorganic material such as a ceramic; a material composed of ceramic and metallic components (cermet); or a polymeric material. The material of construction for the implantable medical device structure can be biodegradable or non-biodegradable. Nonbiodegradable (also referred to as "biodurable") polymers that can be used include for instance cellulose acetate, cellulose nitrate, silicone, polyethylene terephthalate, polyurethane, polyamide, polyester (e.g. Nylon), polyorthoester, polyanhydride, polyether sulfone, polycarbonate, polypropylene, high molecular weight polyethylene, and polytetrafluoroethylene, or mixtures of these. Biodegradable polymers that can be used include for instance polylactic acid (PLA), polyglycolic acid (PGA), poly(lactic-co-glycolic acid) (PLGA), polyanhydride, polycaprolactone, polyhydroxybutyrate valerate, or mixtures of these. Biodegradable metals may also be used, including for example a biodegradable magnesium alloy.

In some preferred embodiments herein, the implantable medical device will be or include a balloon catheter, such as an angioplasty balloon catheter, a scoring balloon catheter or a cutting balloon catheter. Such a balloon catheter can include at least one balloon mounted on a catheter shaft, with the catheter shaft defining an inflation lumen fluidly communicating with an interior of the balloon. The catheter shaft can also define a guide member lumen, for receiving an elongate guidewire or other guiding member for the catheter. The guide member lumen can extend from a distal opening distal to the balloon to a proximal opening proximal to the balloon. The proximal guide member lumen opening can occur in a sidewall of the catheter shaft in a region proximate to the balloon (e.g. within about 10 cm proximal to the proximal end of the balloon) and which is positioned to reside within the patient during use of the balloon catheter, as occurs for example in "rapid-exchange" balloon catheter constructions, or can occur on the catheter shaft in a region positioned to reside external of the patient during use of the balloon catheter, as occurs for example in so-called "over-the-wire" balloon catheter constructions. The balloon catheter may include multiple balloons, usually in this case only two balloons, mounted in positions spaced longitudinally from one another on the catheter shaft. In such cases the balloons may share a common inflation lumen defined by the catheter shaft, or each may have a separate inflation lumen defined by the catheter shaft. In such balloon catheters having only two, or two or more balloons, the distal opening of the guide member lumen can occur distally of the distal-most balloon, and the proximal opening of the guide member lumen can occur proximal of the proximal-most balloon, in either rapid-exchange or over-the-wire type configurations as discussed above. The balloon or balloons of the balloon catheter may carry a TA Release Layer and potentially other coatings, as described herein.

The balloon(s) of the balloon catheters herein may be configured for vascular angioplasty, and/or may have a balloon wall made of any suitable balloon wall material, typically a polymeric balloon wall material. The polymeric or other balloon wall material can be elastomeric, as in the case of an illustrative silicone elastomer, latex rubber elastomer, nylon elastomer, or polyurethane elastomer balloon film, where the balloon can expand upon inflation due to the expansion and thinning of the balloon wall material. The compliance of the balloon wall material in such elastomeric balloon applications is typically greater than 20% and more typically greater than 50%, and/or the burst pressure of such elastomeric balloons will typically be in the range of about 1.1 to about 2 atmospheres. In other embodiments, the polymeric or other balloon wall material can be inelastic, as in the case of a non-compliant or semi-compliant balloon (e.g. as commonly used in angioplasty and/or stent delivery balloons), where the balloon can expand upon inflation due to the unfolding of the balloon wall material from an initial folded configuration. Typical burst pressures for noncompliant and semi-compliant balloons will be in excess of 10 atmospheres, for example in the range of about 10 to about 30 atmospheres. Typical compliance for the balloon wall material in a so-called noncompliant balloon is less than about 10%, and typical compliance for the balloon wall material in a so-called semi-compliant balloon is about 10% to about 20%. Preferred balloon wall materials for non-compliant or semi-compliant balloons include polyamide (e.g. as in Nylon balloons), polyethylene terephthalate (PET), or polyurethane polymers. Preferred non-compliant or semi-compliant balloons suitable for use in vascular vessels such as veins or arteries will include at least a segment that, in an inflated condition of the balloon, defines an elongate, generally cylindrical outer surface configured to contact the wall of the vessel, with the balloon preferably sized to dilate the vessel during such contact. At least a portion of and potentially the entirety of such an elongate, generally cylindrical outer surface can carry a TA Release Layer as discussed herein, either as the sole coating carried by the generally cylindrical outer surface or in combination with one or more additional coatings carried by the generally cylindrical outer surface.

In other preferred embodiments herein, the implantable medical device will be or include a stent. Such a stent may for example be a force-expandable stent, such as a balloon-expandable stent, or a self-expanding stent. The stent may be made from any one of numerous metals and alloys, including those identified hereinabove. The structure of the stent may be formed in a variety of ways to provide a suitable intraluminal support structure having an outer surface for contact with the vessel wall upon implantation and an inner surface that faces the lumen of the vessel and that can be generally opposite the outer surface. For example, the stent may be made from a woven wire structure, a laser-cut cannula, individual interconnected rings, or another pattern or design. In these or other constructions, the stent can include a plurality of struts each having an outer surface for contact with the vessel wall and an inner surface for facing the lumen of the vessel. In certain embodiments the stent may be configured in the form of one or more self-expanding "Z-stents" or Gianturco stents, each of which may comprise a series of substantially straight segments interconnected by a series of bent segments. The bent segments may comprise acute bends or apices. The Gianturco stents are arranged in a zigzag configuration in which the straight segments are set at angles relative to each other and are connected by the bent segments. In other embodiments, the stent may be may be formed from a slotted tube generally comprising a series of longitudinally-adjacent segments and a pattern of connecting segments disposed therebetween. Such stents may be force-expandable, such as balloon-expandable, or self-expanding, as discussed above. Self-expanding stents of this type can be made of a resilient metal, preferably a superelastic metal alloy such as a superelastic nickel-titanium (Ni-Ti) alloy, as occurs for example in the ZILVER® nitinol stent commercially available from Cook Medical, Bloomington, Indiana, USA. Any stent discussed above or elsewhere herein can have a stent surface carrying a TA Release Layer as discussed herein, either as the sole coating carried by the stent surface carrying the TA Release Layer, or in combination with one or more additional coatings positioned underneath and/or overtop the TA Release Layers. As well, surfaces of the stent not carrying a TA Release Layer may optionally be bare (uncoated), or may carry one or more coatings that differ from the TA Release Layer. Additionally, where the stent is mounted on a balloon of a balloon catheter for delivery, the surface of the balloon may carry a TA Release layer and potentially other layer(s) as described herein, and/or the surface of the stent may carry a TA Release layer and potentially other layer(s) as described herein. The practice of these and other variants will be within the purview of those of ordinary skill in the art in view of the teachings herein.

Implantable medical devices of the invention have a TA Release Layer, which includes a (i.e., at least one) water-insoluble therapeutic agent. The term "water-insoluble" as applied to a therapeutic agent herein refers to a therapeutic agent having a solubility in water at 25°C of less than 2 milligrams per milliliter (mg/ml). More preferably, the water-insoluble therapeutic agent has a solubility in water at 25°C of less than 1 mg/ml, even more preferably less than 0.1 mg/ml, and in certain embodiments less than 10 micrograms per milliliter (µg/ml).

The TA Release Layer also includes one or more additives selected from heparin, heparan sulfate, dextran, dextran sulfate, and physiologically-acceptable salts thereof (hereinafter sometimes referred to as "the H/D Additive"). It should be clear that the phrase "one or more additives selected from heparin, heparan sulfate, dextran, dextran sulfate, and physiologically-acceptable salts thereof" and thus "the H/D Additive" includes embodiments wherein each such listed additive is used alone (in the absence of the others) as well as embodiments wherein a mixture of two or more of the additives is used. Thus, the H/D Additive may be: heparin alone or a heparin salt alone; heparan sulfate alone or a heparan sulfate salt alone; dextran alone; dextran sulfate alone or a dextran sulfate salt alone; a mixture of heparin with a heparin salt; a mixture of heparan sulfate with a heparan sulfate salt; a mixture of dextran sulfate with a dextran sulfate salt; a mixture of heparin and/or a salt thereof with heparan sulfate and/or a salt thereof; a mixture of heparin and/or a salt thereof with dextran; a mixture of heparin and/or a salt thereof with and dextran sulfate and/or a salt thereof; a mixture of heparan sulfate and/or a salt thereof with dextran; a mixture of heparan sulfate and/or a salt thereof with dextran sulfate and/or a salt thereof; a mixture of dextran with dextran sulfate and/or a salt thereof; a mixture of heparin and/or a salt thereof, heparan sulfate and/or a salt thereof, and dextran; a mixture of heparin and/or a salt thereof, heparan sulfate and/or a salt thereof, and dextran sulfate and/or a salt thereof; a mixture of heparan sulfate and/or a salt thereof, dextran, and dextran sulfate and/or a salt thereof; or a mixture of heparin and/or a salt thereof, heparan sulfate and/or a salt thereof, dextran, and dextran sulfate and/or a salt thereof. A physiologically-acceptable salt form of heparin, heparan sulfate, and/or dextran sulfate is often used with preference, either alone or in mixtures as noted above, including as examples sodium, potassium and/or calcium salts thereof.

Where a mixture is used as the H/D Additive, one of heparin, heparan sulfate, dextran, dextran sulfate, or a physiologically acceptable salt thereof, may constitute greater than 50% by weight of the H/D Additive, typically greater than about 80%, more typically greater than about 90%, and in preferred embodiments greater than about 95%. An H/D Additive mixture may occur in some instances due to the presence of one or more of the listed additive substances as an impurity in another, typically at a level of less than about 2% by weight.

The therapeutic agent and the H/D Additive, in beneficial embodiments, will constitute greater than 50% by weight of the TA Release Layer, more preferably greater than about 75% by weight, and most preferably greater than about 90% by weight. In certain aspects, the TA Release Layer consists of, or consists essentially of, one or more water-insoluble therapeutic agents (preferably only one) and the H/D Additive. The term "consists essentially of" and its grammatical variants, as applied to a coating of the present invention including the water-insoluble therapeutic agent(s) and the H/D Additive, means that the coating can contain additional components to those specified as long as the additional components do not materially alter the therapeutic effect of the coating. The term "materially alter," as applied to a coating herein, refers to an increase or decrease in the therapeutic effect of the coating of more than about 20% as compared to the effectiveness of a corresponding coating that consists of the water-insoluble therapeutic agent(s) and the H/D Additive. It is contemplated in certain aspects, for example, that by routine experimentation or due to the starting materials used, simple physiologically acceptable inorganic cations, anions or other potentially inert ingredients can be included in the coating, typically in relatively small amounts (e.g. less than about 15% by weight of the coating, more typically less than about 10% by weight), without materially altering the therapeutic effect of the coating.

The term "therapeutic effect" as used herein means an effect which induces, ameliorates or otherwise causes an improvement in the pathological symptoms, disease progression or physiological conditions associated with or resistance to succumbing to a disorder, for example restenosis, of a human or veterinary patient. The term "therapeutically effective amount" as used with respect to a therapeutic agent means an amount of the therapeutic agent which imparts a therapeutic effect to the human or veterinary patient.

Preferred water-insoluble therapeutic agents include water-insoluble antiproliferative agents, immunosuppressive agents, and restenosis-inhibiting agents. In particular embodiments antiproliferative agents or immunosuppressive agents that are restenosis-inhibiting agents are utilized, which can be effective to inhibit restenosis of a vessel when applied to the inner wall of the vessel. In this regard, "restenosis-inhibiting" includes preventing or reducing the extent of restenosis. The inhibition of restenosis may be observed after a procedure in which the vessel wall is injured due to dilatation, for example during dilatation with a balloon of a balloon catheter and/or by expansion of a stent.

In certain aspects of the invention, a water-insoluble restenosis-inhibiting agent is included as a therapeutic agent in the TA Release Layer. The water-insoluble restenosis-inhibiting agent may be the only therapeutic agent in the TA Release Layer, or may be combined with one or more additional therapeutic agents in the TA Release Layer. The water-insoluble restenosis-inhibiting agent may be: a microtubule stabilizing agent such as paclitaxel, a paclitaxel analog, or a paclitaxel derivative or other taxane compound; a macrolide immunosuppressive agent such as sirolimus (rapamycin), pimecrolimus, tacrolimus, everolimus, zotarolimus, novolimus, myolimus, temsirolimus, deforolimus, or biolimus; an antiproliferative agent; a smooth muscle cell inhibitor; an inhibitor of the mammalian target of rapamycin (mTOR inhibitor); or a mixture of two, or two or more of any of these. These or other water-insoluble restenosis-inhibiting agents, including each agent or agent type identified herein, more preferably have a solubility in water at 25°C of less than 1 mg/ml, even more preferably less than 0.1 mg/ml, and in certain embodiments less than 10 micrograms/ml. Paclitaxel, sirolimus, pimecrolimus, tacrolimus, everolimus, zotarolimus, novolimus, myolimus, temsirolimus, deforolimus, and biolimus are preferred water-insoluble restenosis-inhibiting agents for use herein (each known to have a water solubility of less than about 10 micrograms/ml). In certain preferred embodiments, paclitaxel is the only therapeutic agent in the TA Release Layer.

The water-insoluble therapeutic agent can be incorporated in the TA Release Layer at any suitable level. Typically, the water-insoluble therapeutic agent will be incorporated in the TA Release Layer at a level of about 0.0001 to about 1000 micrograms per mm², more typically about 0.01 to about 100 micrograms per mm², and in certain preferred forms about 0.1 to about 10 micrograms per mm². Where two or more therapeutic agents are included in the TA Release Layer, the above-recited levels can apply to the combined weight of all the therapeutic agents, or to the therapeutic agents individually. It will also be understood that the TA Release Layer may contain variations in the level of therapeutic agent in different regions of the coating either due to manufacturing variances or intentional design criteria. Thus, the present invention contemplates TA Release Layers in which the level of therapeutic agent(s) is substantially uniform over the entire area covered by the coating, or in which the level of therapeutic agent(s) differs substantially in one area covered by the TA Release Layer as compared to another area covered by the TA Release Layer. In certain preferred embodiments, paclitaxel is incorporated in the TA Release Layer at a level in the range of about 1 microgram per mm² to about 10 micrograms per mm², more preferably in the range of about 2 micrograms per mm² to about 6 micrograms per mm², either as the only therapeutic agent in the TA Release Layer or in combination with one or more additional therapeutic agents In particularly beneficial implantable medical devices of the invention, such paclitaxel-containing TA Release Layers are carried on a surface of a stent, including for example any stent described herein, and/or on a surface of a balloon of a balloon catheter, including for example any balloon catheter described herein.

The water-insoluble therapeutic agent will typically be incorporated in the TA Release Layer in a therapeutically effective amount. In this regard, it will be understood that where the therapeutic agent is a restenosis-inhibiting agent, the restonosis-inhibiting agent will be incorporated in the coating in an amount that is effective to inhibit restenosis when the implantable medical device (e.g. a balloon or stent) is deployed so as to deliver the therapeutic agent from the TA Release Layer to a wall of the artery, vein or other vessel or passage that is being treated by the implantable medical device. As will be recognized, the level of a therapeutic agent that will be therapeutically effective will vary in accordance with the particular therapeutic agent in use, the implantable medical device in use, the implant site, the condition to be treated, the composition of the coating including the therapeutic agent, and other potential factors. Through routine experimentation in view of the disclosures herein the achievement of a therapeutically effective amount of the water-insoluble therapeutic agent will be within the purview of those of ordinary skilled in the field.

As disclosed above, the therapeutic agent will be incorporated in the TA Release Layer in combination with the H/D Additive. When used as or in the H/D Additive, heparin and/or a heparin salt can have an average molecular weight in the range of about 2000 Daltons to about 40000 Daltons in certain embodiments. More typically, the heparin will have an average molecular weight in the range of about 10000 Daltons to about 30000 Daltons. In this regard, as used herein in reference to heparin or in reference to heparan sulfate, dextran or dextran sulfate, the term "molecular weight" refers to the weight average molecular weight of the composition (M_{w}), as is commonly used by those practicing in the pertinent field.

Heparan sulfate and/or a heparan sulfate salt, when used as or in the H/D Additive, can have a molecular weight M_{w} in the range of about 1000 Daltons to about 70000 Daltons. Those skilled in the field will recognize that heparan sulfate is a sulfated glycosaminoglycan similar to heparin, but is less highly sulfated than heparin. Also, it has been reported that in animals, heparin is produced only by mast cells, whereas heparan sulfate is produced by virtually all cells.

In other embodiments, the TA Release Layer includes dextran and/or dextran sulfate or a salt thereof. Dextran is known as a complex, branched glucan. Dextran can be converted to its derivative dextran sulfate by well-know sulfation techniques. Dextran, dextran sulfate and/or a dextran sulfate salt as used herein can have any suitable molecular weight M_{w}, typically in the range of about 1000 Daltons to about 2000000 Daltons. More preferably, dextran when used will have a molecular weight M_{w} in the range of about 1000 to 10000 Daltons, and dextran sulfate and/or a dextran sulfate salt when used will have a molecular weight M_{w} in the range of about 1000 Daltons to about 1000000 Daltons, more preferably about 1000 Daltons to about 100000 Daltons, and most preferably about 1000 Daltons to about 20000 Daltons.

The H/D Additive can be included in the TA Release Layer in an amount effective to alter the delivery of the water-insoluble therapeutic agent by the TA Release Layer. The weight ratio of the water-insoluble therapeutic agent to the H/D Additive, or to any individual component of the H/D additive, can be in the range of about 20:1 to about 1:1. Preferably, such weight ratio will be in the range of about 20:1 to about 2:1, more preferably in the range of about 15:1 to about 3:1. In further embodiments, such weight ratio will be in the range of about 12:1 to about 7:1, or more specifically in the range of about 12:1 to about 9:1; these weight ratios can be used in particular embodiments where the therapeutic agent is paclitaxel and/or the H/D Additive is heparin alone or a heparin salt alone or is constituted at least 95% by weight of heparin or a heparin salt, with heparin sodium providing a preferred heparin salt for these purposes. In addition or alternatively to the weight ratios noted above, the H/D Additive, or any individual component thereof, can be included in the TA Release Layer at a level of about 0.05 to about 2 micrograms/mm², more preferably about 0.05 to about 1 micrograms/mm², and most preferably about 0.1 to about 1 micrograms/mm² of the TA Release Layer. In these embodiments, the H/D Additive, or any individual component thereof, can be present at a level less than the water-insoluble therapeutic agent.

The H/D Additive can be included in the TA Release Layer in an amount effective to increase the rate of release of the water-insoluble therapeutic agent from the TA Release Layer at a site of implant of the implantable medical device structure. This capacity can be demonstrated, for example, *in in vivo* testing, or in *in vitro* testing where the level of the H/D Additive is observed to increase the rate of release of the water-insoluble therapeutic agent(s) in water, or in an aqueous medium such as blood serum or a 0.2 weight % aqueous solution of Heptakis (2,6-O-methyl)-beta-cyclodextrin (HCD), under static conditions, or stirred conditions, at a temperature of 37°C.

In certain embodiments, the TA Release Layer will be carried by the implantable medical device structure and effective to deliver a therapeutically effective amount of the water-insoluble therapeutic agent to patient tissue in a time period of about 5 minutes or less after implantation of the implantable medical device structure. More preferably, such time period is about 3 minutes or less, even more preferably about 2 minutes or less, and most preferably about 1 minute or less, e.g. in the range of about 20 seconds to about 1 minute. Such coatings configured for relatively rapid delivery are especially beneficial when the TA Release Layer is carried by a surface of a temporarily implantable medical device structure, for example a balloon of a balloon catheter, including any balloon catheter and in any coating arrangement described herein.

It will be understood that in certain embodiments, the TA Release Layer can include ingredients other than water-insoluble therapeutic agent(s) and the H/D Additive. For example, such other ingredients may be included to alter the physical, chemical and/or biologic properties of the TA Release Layer. Illustrative potential additional ingredients include for example ingredients that alter the release of the water-insoluble therapeutic agent(s) from the TA Release Layer and/or that alter the physical stability or adherence of the TA Release Layer to a surface of the implantable medical device or to another coating in turn adhered to the implantable medical device. The additional ingredient(s) in the TA Release Layer may for example be a biodurable polymer; a biodegradable polymer such as polylactic acid (PLA), polyglycolic acid (PGA), poly(lactic-co-glycolic acid) (PLGA), polyanhydride, polycaprolactone, polyhydroxybutyrate valerate, polyethylene glycol (PEG), or a mixture of any or all of these; a contrast agent, such as an iodinated contrast agent, e.g. iobitridol, iohexol, iomeprol, iopamidol, iopentol, iopromide, ioversol, ioxilan, iotrolan, iodixanol, ioxaglate; a molecule having a hydrophilic part and a hydrophobic part, such as a surfactant (e.g. a nonionic surfactant such as a polysorbate surfactant); or one or more water-soluble therapeutic agents; urea; butyryl-tri-hexyl citrate; or a mixture of any or all of these.

Any of a wide variety of coating patterns may be used to constitute a material coat on the medical device. The TA Release Layer can be directly adhered to a surface of an implantable structure of the medical device and provide an outermost surface over the implantable structure, and/or to constitute the entirety of the overall material coat on the implantable structure. In other embodiments, an overall material coat adhered to the implantable structure of the medical device can include one or more different coatings positioned underneath the TA Release Layer (e.g. as in a polymeric or other primer coating, or a different therapeutic agent coating, adhered directly to the surface of the medical device), one or more different coatings positioned overtop the TA Release Layer (e.g. as in a polymeric or other protective or diffusion barrier coating), or both. As well, there may be one or more different coatings adjacent the TA Release Layer, and/or multiple TA Release Layers may be carried by the implantable medical device at locations discrete from one another. The TA Release Layer(s) may occur in an aperture(s) such as a well(s), groove(s) or hole(s) defined in the implantable medical device (e.g. in a stent) or may partially coat or completely coat the implantable medical device or a given surface (e.g. inner, outer or side surface) of the implantable medical device. These and other overall device coating arrangements can be utilized.

The TA Release Layer can be carried by any suitable surface of the implantable medical device structure. The TA Release Layer can be carried by, and in some embodiments only by, a surface or surfaces of the implantable medical device configured for contact with patient tissue when the device is implanted. For example, in some embodiments the TA Release Layer is carried by a surface of a balloon of a balloon catheter, or by a surface of a stent, which is configured for contact with a wall of a vessel when the balloon is implanted (usually temporarily) or when the stent is implanted (usually permanently). In particular embodiments, in the case of a balloon of a balloon catheter which inflates to provide a substantially cylindrical outer surface as discussed above, the TA Release Layer is carried by such substantially cylindrical outer surface, either partially or completely covering the substantially cylindrical surface. In the case of a stent having an outer surface as discussed above, the TA Release Layer can be carried by the outer surface, either partially or completely covering the outer surface.

A first TA Release Layer can be present in combination with another layer including the water-insoluble therapeutic agent without the H/D Additive, or with a second TA Release Layer including a lower H/D Additive:water-insoluble therapeutic agent weight ratio than the first TA Release Layer, such that the other layer or the second TA Release Layer releases the water-insoluble therapeutic agent at a rate slower than the first TA Release Layer. For example, a layer including the water-insoluble therapeutic agent without the H/D Additive can be at least partially overcoated, or undercoated, with a TA Release Layer including the same or another therapeutic agent. In one embodiment, a stent can include a TA Release Layer which at least partially over coats a layer including the water-insoluble therapeutic agent without the H/D Additive. This configuration can allow for the quick delivery of the therapeutic agent from the TA Release Layer followed by a more gradual delivery of the therapeutic agent from the layer without the H/D Additive. The two layers can be separated by one or more layers. Such a configuration can be used in combination with the coating patterns discussed above.

A first TA Release Layer can be present on one surface of an implantable device while another layer including the water-insoluble therapeutic agent without the H/D Additive, or a second TA Release Layer including a lower H/D Additive:water-insoluble therapeutic agent weight ratio than the first TA Release Layer, is present on another surface. For example, a balloon expandable stent and balloon combination can include a stent having a layer including a water-insoluble therapeutic agent without the H/D Additive and a balloon coated with a TA Release Layer including the same or another therapeutic agent. Such a configuration allows for a quicker release dose of the therapeutic agent from the balloon and a slower release of the therapeutic agent from the stent.

The TA Release Layer and any other coating layers present can be incorporated as a part of the implantable medical device by any suitable method. The TA Release Layer and any other coating layer can be formed on a surface of the implantable medical device. For example, the TA Release Layer or other coating layer(s) can be formed by a method that includes dipping, spraying, showering, dripping, or otherwise applying a medium containing the coating ingredients, and optionally a substance such as a solvent can be removed from the medium to leave the coating adhered to the implantable medical device. Spray coating is one preferred form of applying the coating materials to the surface of the implantable medical device, and in particular embodiments ultrasonic spray coating or pressure spray coating will be utilized. During spray coating or other coating operations, the implantable medical device can be moved relative to a sprayer or other applicator of the coating ingredients. This can occur by moving the implantable medical device (including for example rotating the device or at least the portion to be coated), moving the sprayer or other applicator, or both. Multiple application passes or steps will typically be utilized to increase the thickness of the TA Release Layer or other coating layer(s) and control the levels of the water-insoluble therapeutic agent(s), H/D Additive, or other ingredients applied to the implantable medical device. In spray or other application processes, areas of the implantable medical device adjacent to areas desired for coating can optionally be masked to prevent the application of coating materials to the masked areas, and /or portions of applied coating materials can be removed to selectively leave a TA Release Layer or other coating in a desired region or regions of the device.

The water-insoluble therapeutic agent(s) and H/D Additive (and potentially other ingredients) can be combined in a liquid to form a coating medium to be used in the formation of the TA Release Layer on the implantable medical device. This combination can be in the form of a liquid emulsion, suspension, solution, or any other suitable flowable form. Coating mediums provided as solutions are preferred.

A liquid of a solution or other coating medium can in certain aspects contain water, an organic solvent, or a combination thereof. In some embodiments, the coating medium is a solution including the heparin, heparan sulfate, dextran sulfate, dextran, or mixture thereof and the water-insoluble therapeutic agent in an aqueous organic solvent. In these embodiments, the water-insoluble therapeutic agent can have any range of solubility in water for the same discussed hereinabove. A water-insoluble therapeutic agent (e.g. having a solubility in water of less than about 10 micrograms/ml) can be successfully solvated together with heparin, heparan sulfate, dextran sulfate and/or dextran in an aqueous organic solvent, for example having no greater than about 20% by volume water, more preferably no greater than about 10% by volume water, and in certain forms no greater than about 3% by volume water, at levels desirable for applying a coating layer to implantable medical devices, e.g. by spray application, dip application, or otherwise. The organic solvent in such a coating medium is desirably a volatile organic solvent that includes one or a combination of organic compounds that are liquid at room temperature (about 25°C) and at atmospheric pressure (i.e. one atmosphere). The liquid organic compound or compounds may be water-miscible and/or may have from 1 to about 6 carbon atoms. As examples, the liquid organic solvent compound may be an alcohol such as methanol, ethanol, propanol, or butanol; an ester; an ether; a ketone; dimethylsulfoxide; dimethylacetamide; acetonitrile; ethyl acetate; or a mixture of any of these with each other and/or another organic solvent compound.

An aqueous organic solvent solution including the ingredients for the TA Release Layer for use as a coating medium can be prepared in any suitable fashion. In one mode, the H/D Additive is dissolved in water to form an aqueous solution, and the water-insoluble therapeutic agent is dissolved in an organic solvent to form a therapeutic agent solution, and then the aqueous solution is combined with the therapeutic agent solution. This combination is preferably performed under conditions wherein the therapeutic agent and the HD Additive remain solvated in the formed aqueous organic solvent. Slow and/or stepwise addition of the aqueous solution to the therapeutic agent solution is preferred for these purposes.

In certain preparative methods, amounts of a liquid coating medium as described herein can be applied to a surface of an implantable structure of the medical device using a suitable application method (e.g. those discussed above), and the solvent is removed, typically by evaporation, to form a TA Release layer on the surface. The solvent can be caused to evaporate under any suitable conditions therefor, including temperature conditions that may be heated, cooled, or ambient (room temperature), and/or pressure conditions that may be atmospheric (i.e. one atmosphere), greater than atmospheric, or lower than atmospheric, and/or under varied humidity conditions, including reduced humidity conditions (e.g. less than 30%, or less than 20%, humidity).

In other preparative methods, an aqueous solution of the H/D Additive and an organic solvent solution of the water-insoluble therapeutic agent can be combined with one another immediately prior to, or upon, their application to a surface of the implantable structure of the medical device to be coated. Illustratively, in a spray application process, the separate solutions can be fed through separate feed tubes to a spray nozzle, where they can be combined immediately prior to exit from the nozzle. In another spray application process, the aqueous solution including the H/D Additive and the organic solvent therapeutic agent solution can be separately sprayed onto the surface of the medical device to be coated, at or near the same time. The separate solutions can thereby mix, at least to some extent, on the balloon, and the solvent thereafter caused to evaporate, to form the coating.

The TA Release Layer carried by the medical device surface, e.g. formed as described herein, can in certain embodiments be comprised of particulate solids including an admixture including the water-insoluble therapeutic agent and the H/D Additive. Such an admixture is preferably a substantially homogenous admixture. This particulate solids TA Release Layer can have any of the additional physical, compositional, or efficacy characteristics discussed herein. In addition or alternatively, the TA Release Layer can be constituted entirely of material that is releasable from the implantable medical device structure, or may have a biodurable polymer layer attached to the implantable medical device structure and releasably containing the water-insoluble therapeutic agent and H/D Additive, where the biodurable layer remains attached to the device structure as the therapeutic agent and H/D Additive are released. Such a biodurable polymer layer can include a polymeric matrix, e.g. made using a suitable biodurable polymer as identified herein, and in certain forms will be a biodurable porous layer that releasably contains an admixture including the water-insoluble therapeutic agent and H/D Additive in the pores thereof.

As discussed above, in certain embodiments the medical device will have an implantable, expandable portion (e.g. a balloon or stent). In these embodiments the expandable portion can be partially or completely coated with the TA Release Layer either in an expanded condition (including partially or fully expanded), or in a contracted or other delivery condition which typically has a smaller maximum cross sectional profile than the expanded condition. In preferred embodiments, where the medical device has an expandable portion such as a balloon or stent, such expandable portion will be coated in an expanded condition, potentially to include a coating which extends completely around the circumference of the expandable portion (a 360° coat) so that upon deployment of the expandable portion in a vessel, the complete circumference of the vessel can be subjected to delivery of the water-insoluble therapeutic agent by the TA Release Layer. After such coating in the expanded condition, the expandable portion can be converted to a non-expanded configuration. For example, in the case of a balloon of a balloon catheter, after coating with the TA Release Layer in an expanded (e.g. inflated) condition, the balloon can be converted to a folded condition. Such conversion can be conventionally performed, for example by forming pleats from the balloon wall, and radially folding the pleats around the underlying shaft of the balloon catheter. Typically, 2 to 6 pleats are used in these embodiments. Known balloon folding machines can be used for these purposes, which typically include automated tooling that contacts the balloon to form pleats with vacuum assistance applied to the balloon interior, after which the pleats are wrapped radially around the catheter shaft. Suitable such equipment is available, for example, from Machine Solutions, Inc. of Flagstaff Arizona, USA. In the case of an expandable stent, after coating with the TA Release Layer in the expanded condition, the stent can be configured to a smaller profile condition adapted for delivery. In the case of a self-expanding stent, this can be accomplished by radially compressing and thereby resiliently deforming the stent, and potentially loading the stent into a delivery catheter. In the case of a force-expandable stent, this can be accomplished by crimping and thereby plastically deforming the stent, for example around a balloon of a balloon catheter in the case of a balloon-expandable stent. Suitable crimping devices are known for these purposes, and can be used herein.

In certain aspects, a coated medical device as described herein, preferably comprising a stent and/or balloon catheter carrying a TA Release Layer, can be configured to, and used to, treat any suitable body passage in a manner including release of the water-insoluble therapeutic agent to wall tissue of the body passage. The body passage may for example be a vein, artery, biliary duct, ureteral vessel, body passage or portion of the alimentary canal. A coated medical device as described herein may be used to treat a coronary artery, carotid artery, or a peripheral artery or vein, including as examples a renal artery or vein, iliac artery or vein, femoral artery or vein, popliteal artery or vein, subclavian artery or vein, intercranial artery or vein, aorta, vena cava, or others. In preferred embodiments, the coated medical devices will treat or prevent stenosis or restenosis in a body passage such as any of those identified herein, although treatment of other conditions is contemplated for other embodiments of the invention.

In certain embodiments, the coated medical device is configured to, and used to, treat a narrowing of a peripheral artery or vein. Examples of such arteries include, but are not limited to, the femoral artery, the superficial femoral artery (artery below the branch for the profunda femoris artery), the popliteal artery and the infrapopliteal artery. Examples of such veins include, but are not limited to, the femoral vein, the popliteal vein and the lesser/greater saphenous vein.

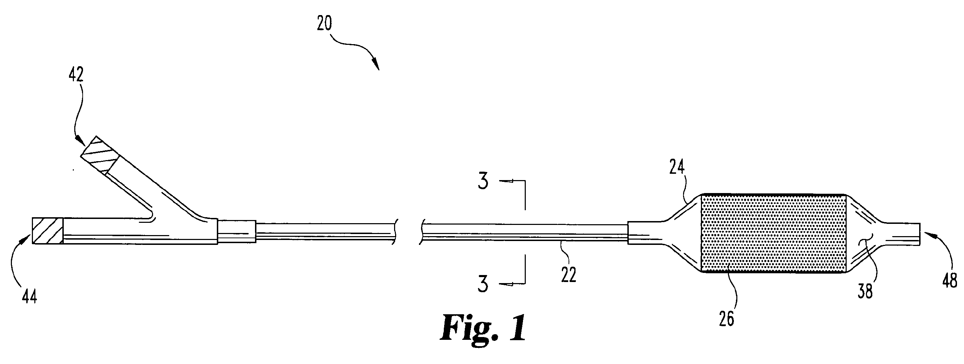

With reference now to **Figs. 1-5**, shown is one embodiment of a therapeutic agent-delivering balloon catheter **20** in accordance with the invention. Balloon catheter **20** includes a catheter shaft **22** and a balloon **24** mounted thereon. A material coat **26** including a TA Release Layer **26a** as described herein is carried by balloon **24.** Catheter shaft **22** includes a first lumen **28** and second lumen **30.** Lumen **28** is configured for inflation of balloon **24**, and lumen **30** is configured to receive a guide wire **32** or other guide member to be used in conjunction with balloon catheter **20.** Balloon **24** includes an interior region **34** designed to receive a liquid or other fluid for inflation of balloon **24.** Balloon **24** has an inner wall **36** bounding balloon interior **34,** and an outer wall surface **38.** TA Release Layer **26** is directly adhered to outer wall surface **38** of balloon **24.**

Balloon catheter **20** also includes a catheter hub **40** mounted to shaft **22.** Catheter hub **40** defines a first opening **42** which fluidly communicates with balloon inflation lumen **28,** and a second opening **44** which fluidly communicates with lumen **30** defined by shaft **22.** Opening **42** of hub **40** and lumen **28** communicate with an opening **46** into the interior **34** of balloon **24,** for passage of the inflation fluid for the balloon **24.** Opening **44** of hub **40** and lumen **30** defined by a catheter shaft **22** extend to distal opening **48** of lumen **30,** with distal opening **48** positioned distally of balloon **24.**

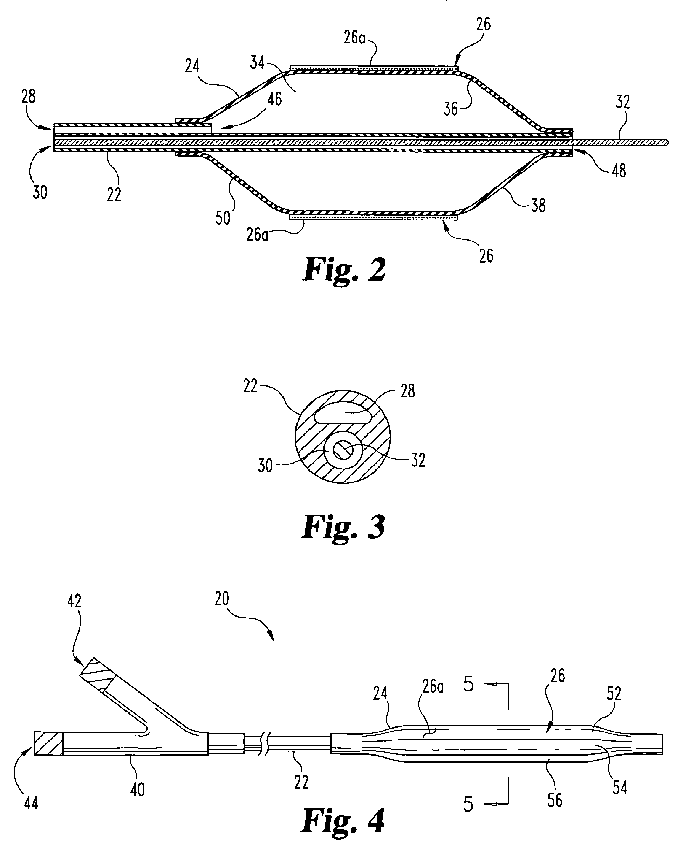

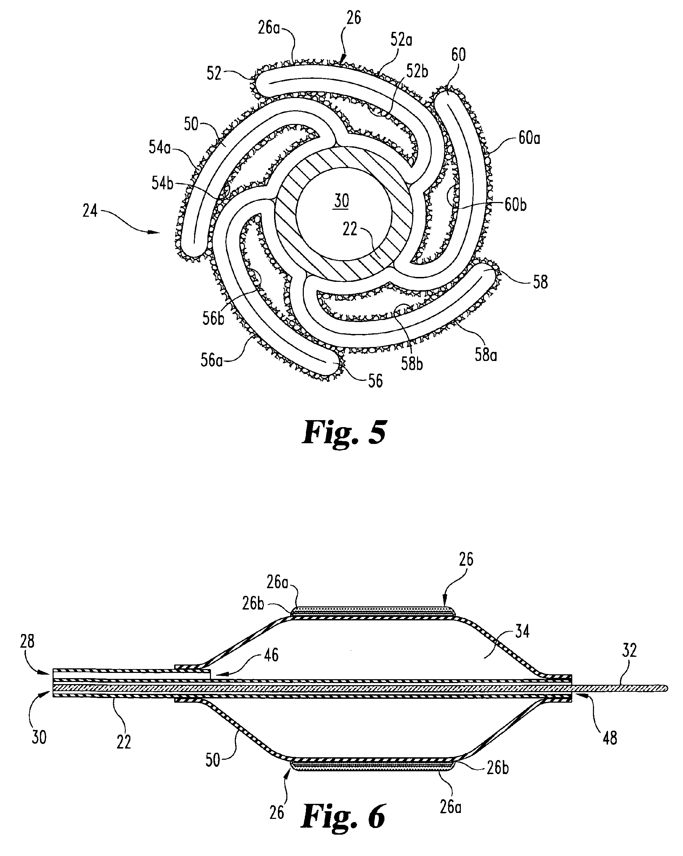

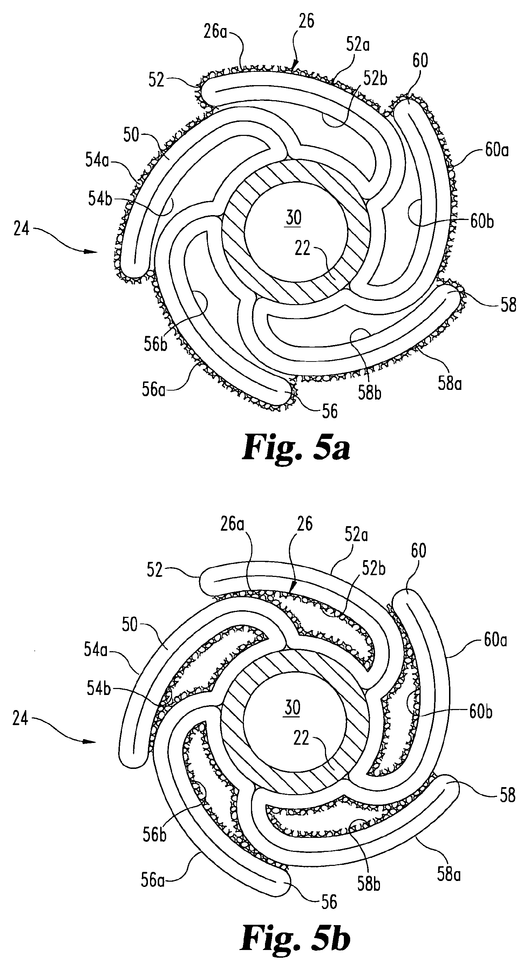

With reference to **Fig.** 5 still in conjunction with features shown in **Figs. 1-****4**, balloon **24** includes a balloon wall **50,** for example defined by a conventional balloon film, typically made from a polymeric material such as one of those discussed hereinabove. Balloon wall **50** as shown in **Figs. 4** and **5** is in a folded condition, useful during insertion of balloon **24** into a vessel such as an artery or vein. In its folded condition, balloon **24** includes pleats **52, 54, 56, 58,** and **60.** As shown, pleats **52-60** are arranged in a spiral pattern with each pleat in a curved condition extending circumferentially around the portion of catheter shaft **22** over which they occur, with the pleats overlapping and thereby contacting one another along at least a portion of their length. In this folded arrangement, pleats **52, 54, 56, 58,** and **60** include externally-exposed pleat surfaces **52a, 54a, 56a, 58a,** and **60a**, and internal non-exposed pleat surfaces **52b**, **54b**, **56b**, **58b**, and **60b**. Correspondingly, material coat **26**, which in the illustrated embodiment includes coating layer **26a**, has externally-exposed portions positioned on externally-exposed pleat surfaces **52a-60a,** and internal non-exposed portions positioned on internal non-exposed pleat surfaces **52b-60b.** Also in this arrangement, because pleats **52-60** overlap with one another, regions of material coat **26** and its TA Release Layer **26a** are in contact with other regions of material coat **26** and its TA Release Layer **26a**. In reference to **Fig. 5****,** it should be understood that the features shown therein are intended to be illustrative, and that in practice balloon **24** is typically tightly pleated and wrapped around catheter shaft **22** and thus there will often be little or no open space on the interior of pleats **52-60**.

Referring now to **Fig. 6****,** shown is another embodiment of a balloon catheter having features similar to those of balloon catheter **20** of **Figs. 1-5****,** but wherein material coat **26** includes a first coating layer **26a**, which is a TA Release Layer as described herein, and a second coating layer **26b** different from the TA Release Layer and positioned underneath coating layer **26a**. Coating layer **26a** may, in certain embodiments, be a polymeric primer layer as discussed above.

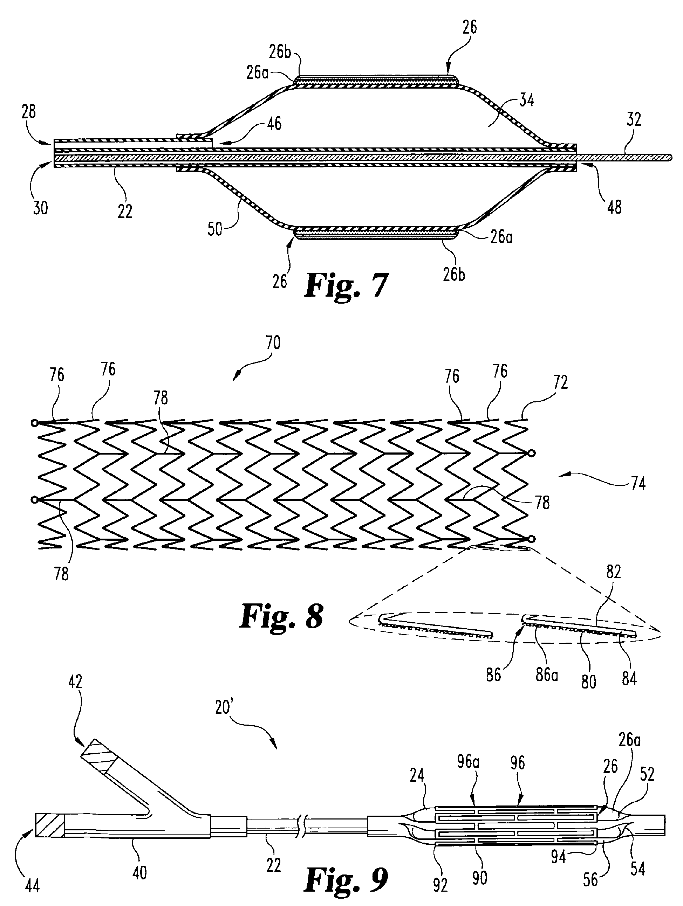

Referring to **Fig. 7****,** shown is another embodiment of a balloon catheter similar to balloon catheter **20** of **Figs. 1-5****,** except wherein material coat **26** includes a first coating layer **26a**, which is a TA Release Layer as described herein, adhered directly to the outer surface **38** of balloon **24,** and a second coating layer **26b** positioned overtop coating layer **26a**. Coating layer **26b** of **Fig.** 7 may, in certain embodiments, be a polymeric protective layer and/or a polymeric diffusion barrier layer operable to control the release of the therapeutic agent(s) through the diffusion barrier layer.

**Figs. 5a** and **5b** show balloon catheter embodiments similar to that shown in **Figs. 1-5** except having a different coating pattern for material coat **26.** In particular, in **Fig. 5a**, the material coat **26** including the TA Release Layer **26a** is carried only by the externally exposed surfaces **52a-60a** of pleats **52-60.** This configuration may be prepared, for example, by coating selected surface areas of the balloon **24** while in the inflated condition that will upon folding be positioned as externally exposed pleat surfaces **52a-60a,** or by coating balloon **24** while in the folded condition under folding and coating conditions that coat only the externally exposed pleat surfaces **52a-60a.** **Fig. 5b** discloses a balloon catheter embodiment in which the material coat **26** is carried only by internal non-exposed pleat surfaces **52b-60b**. This configuration may be prepared, for example, by coating selected surface areas of the balloon **24** while in the inflated condition that will upon folding be positioned as internal non-exposed pleat surfaces **52b-60b**, or by coating balloon **24** completely circumferentially while in the inflated condition, pleating and folding the balloon, and then removing the material coat **26** portions on the externally exposed pleat surfaces **52a-60a,** for example mechanically and/or with a solvent or other medium capable of displacing the material coat **26**. TA Release Layer **26a** of the embodiments of **Figs. 5a** and **5b** can be applied in any suitable fashion, including using any of those methods described herein. As well, the material coat **26** of the embodiments of **Figs. 5a** and **5b** can, in other embodiments, be a multi-layer coating such as those shown and described herein, including those shown and described in conjunction with **Figs. 6** and **7****.**

**Fig. 8** illustrates another embodiment of the invention. A stent **70** includes a stent body **72** defining a central lumen **74.** Stent body **72** includes a plurality of longitudinally-adjacent segments **76** including struts defining a circumferential path about lumen **74** and a pattern of connecting strut segments **78** connecting adjacent segments **76.** A coating **86** (see exploded section, lower right) including a TA Release Layer **86a** is carried by a surface of stent **70.** In the illustrated embodiment, the TA Release Layer **86a** is adhered directly on the surface of stent **72** as the sole coating, although in other embodiments the stent coating **86** may be a multi-layer coating such as those shown and described herein, including those coatings shown and described in conjunction with **Figs. 6** and **7****.** Stent **72** has outer strut surfaces **80** configured for contact with a vessel wall such as an artery or vein wall of a patient. Stent **72** has an inner strut surfaces **82** opposite the outer surfaces **80** and generally facing the lumen **74**. Stent **72** also has a strut sidewall surfaces **84** between outer and inner strut surfaces **80** and **82.** Strut outer surfaces **80** carry the material coat **26** over at least a portion of the outer surface of stent **72** and in certain embodiments over the entire or essentially the entire outer surface of stent **72.** Stent **72** is desirably a self-expanding stent and is preferably made of a resilient metal, preferably a superelastic metal alloy such as a superelastic nickel-titanium (Ni-Ti) alloy, as occurs for example in the ZILVER® nitinol stent commercially available from Cook Medical, Bloomington, Indiana, USA. Stent **72** can be manufactured using methods and materials disclosed herein for stents or otherwise, and the TA Release Layer **26a** and any other coating(s) present on the stent may have any composition taught herein and may be incorporated onto stent **72** in any suitable fashion, including any of those disclosed herein.

**Fig. 9** illustrates another embodiment of the invention. The implantable medical device **20'** of **Fig. 9** is similar to that shown in **Fig. 4****,** except also having a balloon-expandable stent **90** mounted over balloon **24.** Stent **90** has a proximal end **92** and a distal end **94.** In this or other balloon catheters having a stent mounted on the balloon, either the stent **90,** the balloon **24,** or both, can carry a TA Release Layer on a surface thereof. In the illustrated embodiment **20',** the stent **90** has a material coat **96** including a TA Release Layer **96a** carried by an external surface thereof, and the balloon **24** has a material coat **26** including a TA Release Layer **26a** carried by the surface of balloon **24.** The coating layer **26a** can be carried on the balloon **24** so as to extend proximally of proximal end **92** of stent **90** and distally of distal end **94** of stent **90.** In this fashion, the therapeutic agent(s) of the TA Release Layer, when balloon **24** is inflated in a vessel such as an artery or vein to dilate the vessel and implant the stent **90,** can be applied to the vessel in regions extending proximally and distally of the stent **90.** Where the therapeutic agent(s) of the TA Release Layer is or includes a restenosis-inhibiting agent, this can inhibit restenosis that may otherwise occur due to edge effects experienced at or near the proximal **92** and distal **94** ends of the stent **90.**

The balloon and other components of the balloon catheter of device **20',** and the stent **90,** can be manufactured using methods and materials disclosed herein for the same or otherwise. The material coat **26** and/or material coat **96** can include a sole TA Release Layer **26a** or **96a** adhered directly to the surface of balloon **24** or stent **90,** respectively, although in other embodiments the balloon material coat **26** or stent material coat **96** may be a multi-layer coating such as those shown and described herein, including those coatings shown and described in conjunction with **Figs. 6** and **7****.** The TA Release Layer and any other coating layer(s) present on the balloon and/or stent of device **20'** may have any composition taught herein and may be incorporated onto the balloon **24** and/or stent **90** in any suitable fashion, including any of those disclosed herein.

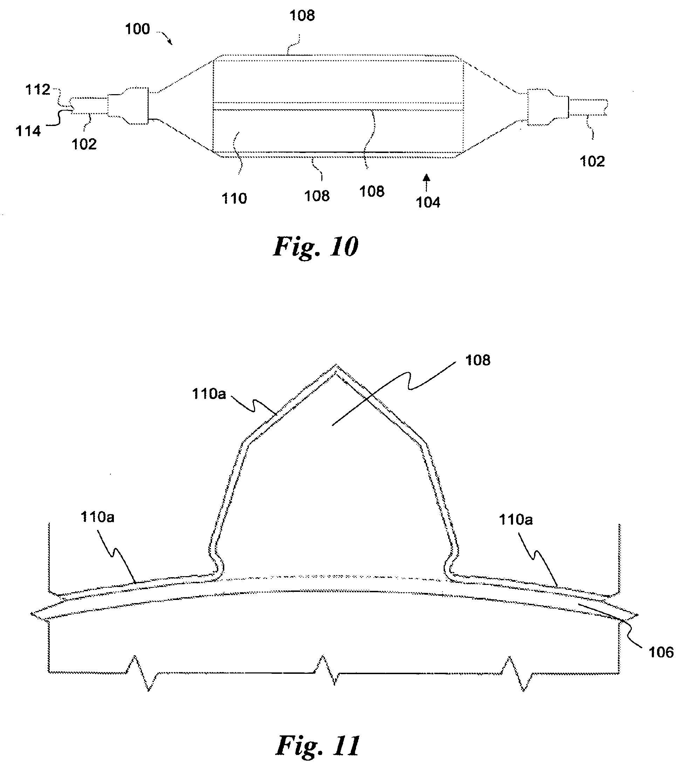

**Figs. 10** and **11** depict another embodiment of the invention. **Fig. 10** provides a side view of a therapeutic agent-delivering scoring balloon according to one embodiment. **Fig. 11** provides an enlarged cross-sectional view of a portion of the balloon of **Fig. 10** including a dilatation element. More specifically, a therapeutic agent-delivering scoring balloon catheter **100** includes a catheter shaft **102** and a balloon **104** mounted thereon. Balloon **104** has attached thereto, and preferably integrally formed with a balloon wall film **106** thereof, a plurality of dilation elements **108** projecting outwardly with respect to the balloon wall film **106** that spans between dilation elements **108.** A material coat **110** including a TA Release Layer **110a** as described herein is carried by balloon **104** and in the specific illustrated embodiment by both the balloon wall film **106** and the dilation elements **108.** Dilation elements **108** as depicted are trizoid-shaped elements; however, other shapes will be suitable for use in embodiments of the present invention, and dilation elements can be provided by separately attached or embedded articles or materials instead of being integrally formed with the balloon wall film. Catheter shaft **102** includes a first lumen **112** and second lumen **114.** Lumen **112** is configured for inflation of balloon **104,** and lumen 114 is configured to receive a guide wire or other guide member to be used in conjunction with balloon catheter **100.** In the embodiment depicted, the TA Release Layer **26a** is directly adhered to outer wall surface of balloon **104** and in particular the outer surface of balloon wall film **106** and dilation elements **108.** It will be understood that in other embodiments, the TA Release Layer can be a part of a material coat that includes multiple layers, including any of those multiple layer coatings described hereinabove, and thus can have other coating layers underneath or overtop the TA Release Layer. In addition or alternatively, the TA Release Layer or material coat incorporating it can extend completely circumferentially around the balloon **104,** coating both the dilation elements **108** and the balloon wall film **106** spanning between the dilation elements **108** (as in **Figs. 10** and **11**), or selective portions of the balloon **104** can be coated. Illustratively, the dilation elements **108** can be completely or partially coated with the TA Release Layer or other material coat including it while the balloon wall film **106** spanning between the dilation elements **108** can be uncoated or at least free of the TA Release Layer or other material coat including it; or, the balloon wall film **106** spanning between the dilation elements **108** can be completely or partially coated with the TA Release Layer or other material coat including it while the dilation elements **108** can be uncoated or at least free of the TA Release Layer or other material coat including it. These and other coating arrangements will be suitable herein. As well, while the balloon 104 is shown in its expanded condition, it will be understood that embodiments herein will include balloon 104 in a folded condition, including for example any of those folded conditions, and structural features provided thereby, described hereinabove.

The following examples illustrate the present invention. The examples and embodiments described herein are for illustrative purposes only and modifications or changes in light thereof will be suggested to one skilled in the art without departing from the scope of the present invention.

### EXAMPLE 1

### Paclitaxel/Heparin Sodium Coated Balloon Catheters

Group A: 10.2 mL water is added to 0.714 g heparin sodium and the mixture is placed in a vortex mixing apparatus until the heparin sodium is dissolved. This solution is added dropwise to a solution of 6 g paclitaxel dissolved in 500 mL ethanol on a shaker table. The resulting solution is a 2% aqueous ethanolic solution containing paclitaxel and heparin sodium in about a 10:1 weight ratio. This solution is fed to a Sonotek ultrasonic coating apparatus and spray-applied to the outer surface of an inflated angioplasty balloon of a Cook Advance® 18LP balloon catheter having a balloon length of 4 cm and an inflated diameter of 7 mm. The coating nozzle is moved relative to the balloon to apply an even layer of an admixture of paclitaxel and heparin sodium sulfate with a dry solids paclitaxel:heparin sodium weight ratio of about 10:1 to the balloon, with the solvent evaporating to form the solids. The spray coating is continued until a coating layer containing approximately 3 µg/mm² of paclitaxel and approximately 0.3 µg/mm² of heparin sodium is formed directly on the balloon wall surface of the balloon.
Group B: Balloon catheters in this group are prepared in the same manner as Group A above, except the coating solution contains the paclitaxel and heparin sodium in a 10:2 weight ratio. The coating layer formed on the balloon wall similarly has a paclitaxel:heparin sodium weight ratio of 10:2 and includes approximately 3 µg/mm² of paclitaxel and approximately 0.6 µg/mm² of heparin sodium.

### EXAMPLE 2

### Dissolution Testing of Paclitaxel/Heparin Sodium Coated Balloon Catheters

Balloon catheters prepared as in Example 1, Group A, were subjected to paclitaxel dissolution testing as compared to balloon catheters similarly prepared except having a paclitaxel-only coating layer containing approximately 3 µg/mm² of paclitaxel. In particular, the coated balloons of the balloon catheters were immersed in a 0.2% weight/volume aqueous solution of Heptakis(2,6-di-O-methyl)-beta-cyclodextrin under static conditions at approximately 37°C. Samples of the dissolution media were taken after various exposure times and assayed to determine the percentage of the paclitaxel originally present on the balloon that had been released. The results evidenced a more rapid release of the paclitaxel from the balloons in the catheters prepared in accordance with Example 1, Group A. After 5 minutes immersion under static conditions, the balloons prepared in accordance with Example 1, Group A (10:1 weight ratio of paclitaxel:heparin sodium) released an average of approximately 0.7% of the original paclitaxel dose present and the paclitaxel-only balloons released an average of approximately 0.4% of the original paclitaxel dose present.

### EXAMPLE 3

### Durability Testing of Paclitaxel/Heparin Sodium Coated Balloon Catheters

Balloon catheters prepared as in Example 1 are subjected to coating layer durability testing as compared to balloon catheters similarly prepared except having a paclitaxel-only coating layer containing approximately 3 µg/mm² of paclitaxel. In particular, the coated balloons of the balloon catheters are passed several times through a water-filled delivery sheath, followed by inflation of the balloon. The coating layer materials remaining on the balloon are thereafter collected in a dissolution medium of 0.5% acetic acid in ethanol which is assayed to determine the percentage of the original paclitaxel dose that remains on the balloon. The data from multiple such runs are averaged, and yield results in which the Group A coated balloons having a paclitaxel:heparin sodium weight ratio of approximately 10:1 demonstrated essentially equivalent durability to the paclitaxel-only balloons, while the Group B coated balloons having a paclitaxel:heparin sodium weight ratio of 10:2 demonstrated a lower durability (and a more rapid release capacity for paclitaxel).

### EXAMPLE 4

### Flow Loop Testing of Paclitaxel/Heparin Sodium Coated Balloon Catheters

Coated balloon catheters prepared in accordance with Example 1, Group A are compared in benchtop flow loop testing to balloon catheters similarly prepared except having a paclitaxel-only coating layer containing approximately 3 µg/mm² of paclitaxel. In particular, excised porcine iliac arteries are arranged in a flow loop with tubing, a pump and a reservoir of porcine serum heated to 37°C to feed the loop. A portion of the loop including the excised artery is immersed in a water bath also maintained at 37°C. Prior to introduction of the coated balloon catheter, flow is established in the loop at a rate of about 510 ml/minute (120 mm Hg pressure). The coated balloon of the catheter is introduced into the loop in the direction of the current flow through a side branch provided by a Y-fitting and advanced into the excised artery. The balloon is then inflated for a period of 60 seconds to contact the coating layer with the artery and dilate the artery, after which the balloon is deflated and the balloon catheter is withdrawn from the loop. The excised artery is then collected, and the treated segment digested with Pronase® (Merck Chemical), and extractions of paclitaxel are taken from the digested tissue in methyl tert-butyl ether and assayed to determine the percentage of the original paclitaxel dose on the balloon that is delivered to the arterial tissue. The results of multiple replicates are averaged yielding an average of about 0.1 % delivery of the original paclitaxel dose of the paclitaxel-only coated balloons and an average of between 0.3% and 0.4% delivery of the original paclitaxel dose of the balloons of Example 1, Group A.

### EXAMPLE 5

### In Vivo Testing of Paclitaxel/Heparin Sodium Coated Balloon Catheters

Coated balloon catheters prepared in accordance with Example 1, Group A are compared in *in vivo* testing in pigs to balloon catheters similarly prepared except having a paclitaxel-only coating layer containing approximately 3 µg/mm² of paclitaxel. In particular, in each test the coated balloon of the catheter is introduced into the iliac artery of a sedated pig through a delivery sheath percutaneously introduced through the carotid artery. The balloon is advanced through the sheath, deployed from the distal opening of the sheath, and then inflated for a period of 60 seconds to contact the coating with the artery and dilate the artery. The balloon catheter and sheath are then withdrawn and the pig immediately sacrificed for immediate collection of the treated segment of iliac artery. The collected tissue segment is then enzymatically digested using Pronase® (Merck Chemical) and the digested tissue is extracted in methyl tert-butyl ether. The extractions are analyzed to determine the percentage of the original paclitaxel dose on the balloon that is delivered to the arterial tissue. The results of multiple replicates are averaged, yielding an average delivered percentage for the balloon catheters of Example 1, Group A that is greater than the average delivered percentage for the paclitaxel-only balloon catheters.

### EXAMPLE 6

### Paclitaxel/Dextran Sulfate Coated Balloon Catheters

Group A: 2100 mg of dextran sodium sulfate (DSS) having a weight average molecular weight M_{w} of 8000 Daltons are dissolved in 3.5 mL water. This solution is slowly added to a solution of 4200 mg paclitaxel dissolved in 70 mL dimethylsulfoxide on a shaker table. 21 mL of ethanol are then added to form a substantially clear solution. The resulting solution is a 3.7% aqueous DMSO-ethanol solution containing paclitaxel and DSS in a 2:1 weight ratio. This solution is fed to a Nordson EFD pressure spray coating apparatus and spray-applied to the outer surface of an inflated angioplasty balloon of a balloon catheter. The coating nozzle is moved relative to the balloon to apply an even layer of an admixture of paclitaxel and DSS with a dry solids paclitaxel:DSS weight ratio of about 2:1to the balloon, with the solvent evaporating to form the solids. Radiant heat is applied in the region of the balloon to speed evaporation of the solvent. The spray coating is continued until a coating layer containing approximately 3 µg/mm² of paclitaxel and approximately 1.5 µg/mm² of DSS is formed directly on the balloon wall surface of the balloon. Balloons thus prepared can be subjected to testing analogous to that described in Examples 2-5. *In vivo testing* similar to that in Example 5 yields results in which an average delivered percentage for the balloon catheters of this Example 6, Group A is greater than the average delivered percentage for corresponding paclitaxel-only balloon catheters.
Group B: 30 mg of dextran sodium sulfate (DSS) having a weight average molecular weight M_{w} of 8000 Daltons are dissolved in 1 mL water. This solution is slowly added to a solution of 180 mg paclitaxel dissolved in 10 mL ethanol on a shaker table. The resulting solution is a 9% aqueous ethanol solution containing paclitaxel and DSS in a 6:1 weight ratio. This solution is fed to a Nordson EFD pressure spray coating apparatus and spray-applied to the outer surface of an inflated angioplasty balloon of a balloon catheter. Prior to coating, the surface of the balloon is plasma cleaned. The coating nozzle is moved relative to the balloon to apply an even layer of an admixture of paclitaxel and DSS with a dry solids paclitaxel:DSS weight ratio of about 6:1 to the balloon, with the solvent evaporating to form the solids. The spray coating is continued until a coating layer containing approximately 3 µg/mm² of paclitaxel and approximately 0.5 µg/mm² of DSS is formed directly on the balloon wall surface of the balloon. Balloons thus prepared can be subjected to testing analogous to that described in Examples 2-5.

### EXAMPLE 7

### Paclitaxel/Dextran Coated Balloon Catheters

20 mg of dextran having a weight average molecular weight M_{w} of about 1500 are dissolved in 3 mL water. This solution is slowly added to a solution of 120 mg paclitaxel dissolved in 20 mL ethanol on a shaker table. The resulting solution is a 13% aqueous ethanol solution containing paclitaxel and dextran in a 6:1 weight ratio. This solution is fed to a Nordson EFD pressure spray coating apparatus and spray-applied to the outer surface of an inflated angioplasty balloon of a balloon catheter. The coating nozzle is moved relative to the balloon to apply an even layer of an admixture of paclitaxel and dextran with a dry solids paclitaxel:dextran weight ratio of about 6:1to the balloon, with the solvent evaporating to form the solids. The spray coating is continued until a coating layer containing approximately 3 µg/mm² of paclitaxel and approximately 0.5 µg/mm² of DSS is formed directly on the balloon wall surface of the balloon. Balloons thus prepared can be subjected to testing analogous to that described in Examples 2-5.

The uses of the terms "a" and "an" and "the" and similar references in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention.

## Claims

1. A medical device, comprising:
an implantable medical device structure having a surface; and
a coating layer carried by the surface and including:
(i) a water-insoluble therapeutic agent having a solubility in water at 25°C of less than 2 milligrams per milliliter; and
(ii) one or more additives selected from heparin, heparan sulfate, dextran, and dextran sulfate; and physiologically-acceptable salts thereof;
wherein the water-insoluble therapeutic agent is included in the coating layer in a weight ratio in the range of about 20:1 to about 2:1 relative to said one or more additives.

2. The medical device of claim 1, wherein the implantable medical device structure is an expandable structure.

3. The medical device of claim 2, wherein the expandable structure is a balloon, preferably wherein the balloon is an angioplasty balloon.

4. The medical device of claim 2, wherein the expandable structure is a stent.

5. The medical device of any preceding claim, wherein the water-insoluble therapeutic agent is an immunosuppressive agent, an antiproliferative agent, a microtubule stabilizing agent, a restenosis-inhibiting agent, or an inhibitor of the mammalian target of rapamycin.

6. The medical device of any preceding claim, wherein the water-insoluble therapeutic agent is a taxane compound.

7. The medical device of claim 8, wherein the taxane compound is paclitaxel.

8. The medical device of any preceding claim, wherein the coating layer is comprised of particulate solids including an admixture including the water-insoluble therapeutic agent and the one or more additives.

9. The medical device of any preceding claim, wherein the one or more additives includes heparin.

10. The medical device of any preceding claim, wherein the one or more additives is present in an amount effective to increase the rate of release of the water-insoluble therapeutic agent from the coating layer.

11. The medical device of any preceding claim, wherein the one or more additives includes heparin or a physiologically-acceptable salt thereof, having a weight average molecular weight in the range of about 2000 to about 40000 Daltons.

12. The medical device of any preceding claim, wherein the one or more additives includes heparin sodium.

13. The medical device of any preceding claim, wherein:
the water-insoluble therapeutic agent is paclitaxel;
the coating layer comprises the paclitaxel at a level of about 1 micrograms/mm² to about 10 micrograms/mm²; and
the coating layer comprises heparin sodium at a level less than the paclitaxel and in the range of about 0.05 to about 2 micrograms/mm².

14. A method for manufacturing a medical device, comprising:
applying a flowable medium comprising liquid, a water-insoluble therapeutic agent having a solubility in water at 25°C of less than 2 milligrams per milliliter and one or more additives selected from heparin, heparan sulfate, dextran and dextran sulfate, and physiologically-acceptable salts thereof, to a surface of an implantable medical device structure or to a surface of a coating layer carried by the implantable medical device structure, wherein the water-insoluble therapeutic agent is present in the flowable medium in a weight ratio in the range of about 20:1 to about 2:1 with respect to the one or more additives; and
removing liquid from the medium to form a coating layer comprising the water-insoluble therapeutic agent and the one or more additives.

15. The method of claim 14, wherein the implantable medical device structure is an expandable structure, and preferably wherein the implantable medical device structure is a balloon.

16. The method of claim 14 or 15, wherein the water-insoluble therapeutic agent is a taxane.

17. The method of claim 16, wherein the taxane is paclitaxel.

18. The method of any one of claims 15 to 17, wherein the one or more additives is present in an amount effective to increase the rate of release of the water-insoluble therapeutic agent from the coating layer.

19. A coating layer including (i) a water-insoluble therapeutic agent having a solubility in water at 25°C of less than 2 milligrams per milliliter and (ii) one or more additives selected from heparin, heparan sulfate, dextran and dextran sulfate, and physiologically acceptable salts thereof, wherein the water-insoluble therapeutic agent is included in the coating layer in a weight ratio in the range of about 20:1 to about 2:1 relative to said one or more additives, for delivery of the water-insoluble therapeutic agent from an implantable medical device structure.

## Patentansprüche

1. Medizinische Vorrichtung, umfassend:
eine implantierbare medizinische Vorrichtungsstruktur mit einer Oberfläche; und
eine von der Oberfläche getragene Beschichtungsschicht, umfassend:
(i) ein wasserunlösliches therapeutisches Mittel mit einer Löslichkeit in Wasser bei 25 °C von weniger als 2 Milligramm pro Milliliter; und
(ii) einen oder mehrere Zusatzstoffe ausgewählt aus Heparin, Heparansulfat, Dextran und Dextransulfat; und physiologisch annehmbaren Salzen davon;
wobei das wasserunlösliche therapeutische Mittel in einem Gewichtsanteil in dem Bereich von etwa 20:1 bis etwa 2:1 bezogen auf den einen oder die mehreren Zusatzstoffen in der Beschichtungsschicht enthalten ist.

2. Medizinische Vorrichtung gemäß Anspruch 1, wobei die implantierbare medizinische Vorrichtungsstruktur eine expandierbare Struktur ist.

3. Medizinische Vorrichtung gemäß Anspruch 2, wobei die expandierbare Struktur ein Ballon ist, vorzugsweise wobei der Ballon ein Angioplastieballon ist.

4. Medizinische Vorrichtung gemäß Anspruch 2, wobei die expandierbare Struktur ein Stent ist.

5. Medizinische Vorrichtung gemäß einem der vorstehenden Ansprüche, wobei das wasserunlösliche therapeutische Mittel ein immunsuppressives Mittel, ein antiproliferatives Mittel, ein Mikrotubulus-stabilisierendes Mittel, ein Restenose-hemmendes Mittel oder ein Hemmer des Säugerziels von Rapamycin ist.

6. Medizinische Vorrichtung gemäß einem der vorstehenden Ansprüche, wobei das wasserunlösliche therapeutische Mittel eine Taxanverbindung ist.

7. Medizinische Vorrichtung gemäß Anspruch 8, wobei die Taxanverbindung Paclitaxel ist.

8. Medizinische Vorrichtung gemäß einem der vorstehenden Ansprüche, wobei die Beschichtungsschicht aus partikelförmigen Feststoffen besteht, umfassend ein Gemisch umfassend das wasserunlösliche therapeutische Mittel und den einen oder die mehreren Zusatzstoffe.

9. Medizinische Vorrichtung gemäß einem der vorstehenden Ansprüche, wobei der eine oder die mehreren Zusatzstoffe Heparin umfassen.

10. Medizinische Vorrichtung gemäß einem der vorstehenden Ansprüche, wobei der eine oder die mehreren Zusatzstoffe in einer Menge vorhanden sind, die wirksam ist, die Freisetzungsrate des wasserunlöslichen therapeutischen Mittels aus der Beschichtungsschicht zu erhöhen.

11. Medizinische Vorrichtung gemäß einem der vorstehenden Ansprüche, wobei der eine oder die mehreren Zusatzstoffe Heparin oder ein physiologisch annehmbares Salz davon mit einem gewichtsgemittelten Molekulargewicht in dem Bereich von etwa 2000 bis etwa 40000 Dalton umfassen.

12. Medizinische Vorrichtung gemäß einem der vorstehenden Ansprüche, wobei der eine oder die mehreren Zusatzstoffe Heparin-Natrium umfassen.

13. Medizinische Vorrichtung gemäß einem der vorstehenden Ansprüche, wobei:
das wasserunlösliche therapeutische Mittel Paclitaxel ist;
die Beschichtungsschicht das Paclitaxel in einer Menge von etwa 1 Mikrogramm/mm² bis etwa 10 Mikrogramm/mm² umfasst; und
die Beschichtungsschicht Heparin-Natrium in einer Menge von weniger als das Paclitaxel und in dem Bereich von etwa 0,05 bis etwa 2 Mikrogramm/mm² umfasst.

14. Verfahren zum Herstellen einer medizinischen Vorrichtung, umfassend:
Aufbringen eines fließfähigen Mediums, das Flüssigkeit, ein wasserunlösliches therapeutisches Mittel mit einer Löslichkeit in Wasser bei 25 °C von weniger als 2 Milligramm pro Milliliter und einen oder mehrere Zusatzstoffe ausgewählt aus Heparin, Heparansulfat, Dextran und Dextransulfat und physiologisch annehmbaren Salzen davon umfasst, auf eine Oberfläche einer implantierbaren medizinischen Vorrichtungsstruktur oder auf eine Oberfläche einer Beschichtungsschicht, die von der implantierbaren medizinischen Vorrichtungsstruktur getragen wird, wobei das wasserunlösliche therapeutische Mittel in dem fließfähigen Medium in einem Gewichtsanteil in dem Bereich von etwa 20:1 bis etwa 2:1 bezogen auf den einen oder die mehreren Zusatzstoffe enthalten ist; und
Entfernen von Flüssigkeit aus dem Medium, um eine Beschichtungsschicht zu bilden, die das wasserunlösliche therapeutische Mittel und den einen oder die mehreren Zusatzstoffe umfasst.

15. Verfahren gemäß Anspruch 14, wobei die implantierbare medizinische Vorrichtungsstruktur eine expandierbare Struktur ist und vorzugsweise wobei die implantierbare medizinische Vorrichtungsstruktur ein Ballon ist.

16. Verfahren gemäß Anspruch 14 oder 15, wobei das wasserunlösliche therapeutische Mittel ein Taxan ist.

17. Verfahren gemäß Anspruch 16, wobei das Taxan Paclitaxel ist.

18. Verfahren gemäß einem der Ansprüche 15 bis 17, wobei der eine oder die mehreren Zusatzstoffe in einer Menge vorhanden sind, die wirksam ist, die Freisetzungsrate des wasserunlöslichen therapeutischen Mittels aus der Beschichtungsschicht zu erhöhen.

19. Beschichtungsschicht, umfassend (i) ein wasserunlösliches therapeutisches Mittel mit einer Löslichkeit in Wasser bei 25 °C von weniger als 2 Milligramm pro Milliliter und (ii) einen oder mehrere Zusatzstoffe ausgewählt aus Heparin, Heparansulfat, Dextran und Dextransulfat und physiologisch annehmbaren Salzen davon, wobei das wasserunlösliche therapeutische Mittel in einem Gewichtsanteil in dem Bereich von etwa 20:1 bis etwa 2:1 bezogen auf den einen oder die mehreren Zusatzstoffen in der Beschichtungsschicht enthalten ist, für die Abgabe des wasserunlöslichen therapeutischen Mittels aus einer implantierbaren medizinischen Vorrichtungsstruktur.

## Revendications

1. Dispositif médical, comprenant :
une structure de dispositif médical implantable comportant une surface ; et
une couche de revêtement portée par la surface et incluant :
(i) un agent thérapeutique insoluble dans l'eau présentant une solubilité dans l'eau à 25 °C de moins de 2 milligrammes par millilitre ; et
(ii) un ou plusieurs adjuvants choisis parmi l'héparine, le sulfate d'héparane, le dextrane et le sulfate de dextrane ; et leurs sels physiologiquement acceptables ;
où l'agent thérapeutique insoluble dans l'eau est inclus dans la couche de revêtement dans un rapport massique compris entre environ 20:1 et environ 2:1 par rapport audit adjuvant ou auxdits adjuvants.

2. Dispositif médical selon la revendication 1, où la structure de dispositif médical implantable est une structure expansible.

3. Dispositif médical selon la revendication 2, où la structure expansible est un ballonnet, préférentiellement où le ballonnet est un ballonnet d'angioplastie.

4. Dispositif médical selon la revendication 2, où la structure expansible est un stent.

5. Dispositif médical selon l'une quelconque des revendications précédentes, où l'agent thérapeutique insoluble dans l'eau est un agent immunosuppresseur, un agent antiproliférant, un agent stabilisant les microtubules, un agent inhibant la resténose ou un inhibiteur de la cible mammalienne de la rapamycine.

6. Dispositif médical selon l'une quelconque des revendications précédentes, où l'agent thérapeutique insoluble dans l'eau est un taxane.

7. Dispositif médical selon la revendication 8, où le taxane est le paclitaxel.

8. Dispositif médical selon l'une quelconque des revendications précédentes, où la couche de revêtement est composée de particules solides incluant un mélange incluant l'agent thérapeutique insoluble dans l'eau et le ou les adjuvants.

9. Dispositif médical selon l'une quelconque des revendications précédentes, où le ou les adjuvants incluent l'héparine.

10. Dispositif médical selon l'une quelconque des revendications précédentes, où le ou les adjuvants sont présents en une quantité permettant d'augmenter la vitesse de libération de l'agent thérapeutique insoluble dans l'eau depuis la couche de revêtement.

11. Dispositif médical selon l'une quelconque des revendications précédentes, où le ou les adjuvants incluent l'héparine ou l'un des sels physiologiquement acceptables de celle-ci, de masse moléculaire moyenne en poids comprise entre environ 2000 et environ 40 000 daltons.

12. Dispositif médical selon l'une quelconque des revendications précédentes, où le ou les adjuvants incluent l'héparine sodium.

13. Dispositif médical selon l'une quelconque des revendications précédentes, où :
l'agent thérapeutique insoluble dans l'eau est le paclitaxel ;
la couche de revêtement comprend le paclitaxel à une teneur comprise entre environ 1 microgramme/mm² et environ 10 microgrammes/mm² ; et
la couche de revêtement comprend de l'héparine sodium à une teneur inférieure au paclitaxel et comprise entre environ 0,05 et environ 2 microgrammes/mm².

14. Procédé de fabrication d'un dispositif médical comprenant :
l'application d'un milieu fluide comprenant un liquide, un agent thérapeutique insoluble dans l'eau présentant une solubilité dans l'eau à 25 °C de moins de 2 milligrammes par millilitre et un ou plusieurs adjuvants choisis parmi l'héparine, le sulfate d'héparane, le dextrane et le sulfate de dextrane, ainsi que leurs sels physiologiquement acceptables, sur une surface d'une structure de dispositif médical implantable ou sur une surface d'une couche de revêtement portée par la structure de dispositif médical implantable, où l'agent thérapeutique insoluble dans l'eau est présent dans le milieu fluide dans un rapport massique compris entre environ 20:1 et environ 2:1 par rapport à l'adjuvant ou aux adjuvants ; et l'élimination du liquide depuis le milieu pour former une couche de revêtement comprenant l'agent thérapeutique insoluble dans l'eau et le ou les adjuvants.

15. Procédé selon la revendication 14, où la structure de dispositif médical implantable est une structure expansible, et préférentiellement où la structure de dispositif médical implantable est un ballonnet.

16. Procédé selon la revendication 14 ou 15, où l'agent thérapeutique insoluble dans l'eau est un taxane.

17. Procédé selon la revendication 16, où le taxane est le paclitaxel.

18. Procédé selon l'une quelconque des revendications 15 à 17, où le ou les adjuvants sont présents en une quantité permettant d'augmenter la vitesse de libération de l'agent thérapeutique insoluble dans l'eau depuis la couche de revêtement.

19. Couche de revêtement incluant (i) un agent thérapeutique insoluble dans l'eau présentant une solubilité dans l'eau à 25 °C de moins de 2 milligrammes par millilitre et (ii) un ou plusieurs adjuvants choisis parmi l'héparine, le sulfate d'héparane, le dextrane et le sulfate de dextrane, ainsi que leurs sels physiologiquement acceptables, où l'agent thérapeutique insoluble dans l'eau est présent dans la couche de revêtement dans un rapport massique compris entre environ 20:1 et environ 2:1 par rapport audit adjuvant ou auxdits adjuvants, pour libération de l'agent thérapeutique insoluble dans l'eau à partir d'une structure de dispositif médical implantable.
